# EUROPÄISCHE PATENTSCHRIFT

(11) **EP 4 122 434 B1**
(45) Veröffentlichungstag und Bekanntmachung des Hinweises auf die Patenterteilung: **29.10.2025**
(21) Anmeldenummer: 21187427.6
(22) Anmeldetag: 23.07.2021
(51) Int. Cl.: D01F 1/10, D01D 5/00, A61F 11/00

(54) **TEXTILES FLÄCHENGEBILDE UND VORRICHTUNG ZUR VERWENDUNG ALS THERAPEUTISCHES MITTEL**
TEXTILE FABRIC AND DEVICE FOR USE AS A THERAPEUTIC AGENT
STRUCTURE PLANE TEXTILE ET DISPOSITIF DESTINÉ À ÊTRE UTILISÉ COMME MOYEN THÉRAPEUTIQUE

(43) Veröffentlichungstag der Anmeldung: 25.01.2023
(73) Patentinhaber: Universitätsklinikum Jena, 07747 Jena (DE); Innovent e.V., 07745 Jena (DE)
(72) Erfinder: PLETZ, Mathias, 31535 Neustadt a. Rbge (DE); WYRWA, Ralf, 07715 Rothenstein (DE)
(74) Vertreter: Liedtke & Partner Patentanwälte

(56) Entgegenhaltungen:
- CH-A2- 699 879
- DE-A1- 10 049 068
- DE-A1- 3 743 104
- ARENBERGEROVA MONIKA ET AL: "Light-activated nanofibre textiles exert antibacterial effects in the setting of chronic wound healing", EXPERIMENTAL DERMATOLOGY, vol. 21, no. 8, 1 August 2012 (2012-08-01), COPENHAGEN; DK, pages 619 - 624, XP055877057, ISSN: 0906-6705, DOI: 10.1111/j.1600-0625.2012.01536.x
- BOZJA J ET AL: "Porphyrin-Based, Light-Activated Antimicrobial Materials", JOURNAL OF POLYMER SCIENCE PART A: POLYMER CHEMISTRY, JOHN WILEY & SONS, INC, US, vol. 41, 1 January 2003 (2003-01-01), pages 2297 - 2303, XP002535483, ISSN: 0887-624X, DOI: 10.1002/POLA.10773

## Beschreibung

Die Erfindung betrifft eine Vorrichtung zur Verwendung bei einer Behandlung. Des Weiteren betrifft die Erfindung ein Verfahren zur Herstellung der Vorrichtung zur Verwendung bei einer Behandlung.

Es ist bekannt, dass eine Entzündung eines Gehörgangs (Otitis externa), insbesondere eines äußeren Gehörgangs bzw. Ohres äußerst unangenehm ist. In einer beispielsweise feuchten, akuten Form ist die Entzündung gekennzeichnet durch eine sehr schmerzhafte Schwellung des Gehörgangs bzw. des äußeren Ohres mit beispielsweise schmieriger Sekretion, Schuppenbildung und/oder Juckreiz.

Erreger der Entzündung sind beispielsweise häufig in Badewassern zu finden.

Im Stand der Technik sind Behandlungsverfahren bekannt, bei welchen beispielsweise ein mit Alkohol konzentrierter oder mit speziellen Ohrentropfen getränkter Mullstreifen am und/oder im Gehörgang eingelegt wird. Alternativ werden zur Behandlung der Entzündung, beispielsweise bei Kindern, Ohrentropfen verabreicht. Nachteilig hierbei ist, dass sich Ohrentropfen ungleichmäßig und unkontrolliert verteilen können. Die Ohrentropfen umfassen meist Antibiotika und/oder entzündungshemmende Wirkstoffe.

Aus dem Artikel "Light-activated nanofiber textiles exert antibacterial effects in the setting of chronic wound healing", Arenbergerova Monika et al., Experimental Dermatology, Bd. 21, Nr. 8, 1. August 20212, Seiten 619-624, (ISSN: 0906-6705, DOI: 10.1111/j.1600-0625.2012.01536x) ist ein textiles Flächengebilde zur Verwendung als therapeutisches Mittel zur Behandlung von Hautkrankheiten bekannt, wobei das textile Flächengebilde mit einer gasfreisetzenden Substanz versehen ist, welche bei Aktivierung durch Licht als antibakterielles Gas Sauerstoffradikale freisetzt.

Aus CH699 879 A2 ist eine Verabreichung von flüssigen Wirkstoffen in den Gehörgang bekannt.

Der Erfindung liegt die Aufgabe zu Grunde, eine Vorrichtung zur Verwendung bei einer Behandlung anzugeben, welche eine einfache Eradikation von Bakterien ermöglicht. Des Weiteren ist ein besonders einfaches Verfahren zur Herstellung der Vorrichtung zur Verwendung bei einer Behandlung anzugeben.

Hinsichtlich der Vorrichtung wird die Aufgabe erfindungsgemäß durch die Merkmale des Patentanspruchs 1 gelöst. Hinsichtlich des Verfahrens wird die Aufgabe erfindungsgemäß durch die Merkmale des Patentanspruchs 12 gelöst.

Vorteilhafte Ausgestaltungen der Erfindung sind Gegenstand der Unteransprüche.

Die Vorrichtung umfasst ein textiles Flächengebilde zur Verwendung als therapeutisches Mittel, wobei das textile Flächengebilde mit zumindest einer Gas-freisetzenden oder Therapeutikum-freisetzenden Substanz versehen ist, welche bei Aktivierung durch Licht ein antibakterielles Gas freisetzt.

Beispielsweise gelangen Bakterien in Körperöffnungen oder -höhlen, wie zum Beispiel in Gehörgänge eines Menschen. Bei einer so genannten Otitis externa handelt es sich um eine Entzündung des äußeren Gehörganges, die oftmals durch ein gramnegatives, oxidasepositives Stäbchenbakterium der Gattung Pseudomonas (Pseudomonas aeruginosa) hervorgerufen wird. Dieser Erreger findet sich häufig in Badewassern. Beispielsweise sind insbesondere Kinder von einer solchen Infektion betroffen. Aufgrund seiner intrinsinischen Resistenz ist es bisher bekannt für Pseudomonas aeruginosa, nur Fluorchinolonantibiotikum (Fluorchinolone) als oral verfügbare Antibiotika zu verabreichen. Orale Fluorochinolon-Antibiotika sind bei Kindern aufgrund einer potentiellen Schädigung des Knorpelwachstums kontraindiziert. Somit beschränkt sich eine Therapiemöglichkeit der Otitis externa bei Kindern, insbesondere der so genannten Badeotitis, auf Fluorchinolon-Ohrentropfen. Dabei ist eine ungleichmäßige Verteilung der Ohrentropfen im betroffenen äußeren Gehörgang problematisch, wobei eine effektive und gezielte Behandlung schwer kontrollierbar ist.

Vorteile der Erfindung sind eine einfache Handhabe und ein einfacher Aufbau des textilen Flächengebildes sowie eine im Wesentlichen schmerzfreie Behandlung von zum Beispiel entzündeter Körperhöhlen oder -öffnungen sowie die Wirkung auf Bakterien unabhängig vom Vorliegen etwaiger Antibiotikaresistenzen. Beispielsweise handelt es sich bei der Körperhöhle um einen Gehörgang, insbesondere einen äußeren Gehörgang oder von außen zugängliche Abszesshöhlen nach Entlastung und Drainage des Eiters oder eine andere Körperöffnung. Derartige Körperöffnungen können zusätzlich vergleichsweise leicht verschlossen werden. Somit kann eine hohe lokale Konzentration des antibakteriellen Gases erreicht werden, ohne dass beispielsweise ein Übertritt in einen Blutstrom, eine CO-Hämoglobinämie und/oder Toxizität befürchtet werden muss.

Das textile Flächengebilde wird als therapeutisches Mittel zur Eradikation von Bakterien in Körperhöhlen verwendet, wobei insbesondere zumindest ein antibakterielles Gas in eine abgedichtete Körperhöhle freigesetzt wird, in welcher das textile Flächengebilde angeordnet ist.

Dabei setzt die Gas-freisetzende Substanz bei Aktivierung ein antibakteriell wirkendes Gas, zum Beispiel Kohlenstoffmonoxid oder Stickstoffmonoxid, frei. Es hat sich herausgestellt, dass als Gas-freisetzendes Molekül ein Carbonylkomplex eines Übergangsmetalls vorteilhaft ist. Besonders vorteilhaft umfasst das Gas-freisetzende Molekül zumindest Mn₂(CO)₁₀. Alternativ können auch Nitrosylkomplexe als Gas-freisetzende Moleküle verwendet werden. Ebenso sind Mischungen aus CO- und NO-freisetzenden Substanzen einsetzbar.

In einer Ausführungsform ist das textile Flächengebilde aus Stoffvlies gebildet. Beispielsweise ist das textile Flächengebilde vollständig oder teilweise aus Stoffvlies gebildet. Das textile Flächengebilde kann aus Fasern gebildet sein, die zur Aufnahme und Lagerung einer Gas-freisetzenden Substanz oder von Gas-freisetzenden Molekülen geeignet sind. Insbesondere umfasst Vlies solche Eigenschaften. Des Weiteren ist Vlies für seine Eigenschaften wie Strapazierfähigkeit, Formstabilität, Reißfestigkeit, Wirkstoffaufnahme und - abgabe sowie Nachgiebigkeit bekannt. Daher ist das textile Flächengebilde aus Stoffvlies einfach als therapeutisches Mittel verwendbar. Das textile Flächengebilde wird insbesondere als therapeutisches Mittel bei einer Behandlung einer entzündlichen Erkrankung in einer Körperhöhle verwendet. Die entzündliche Erkrankung ist beispielsweise eine Entzündung des äußeren Gehörganges (Otitis externa) oder eine von außen zugängliche drainierte Abszesshöhle. Dabei kann das Stoffvlies im Wesentlichen schmerzfrei in die Körperhöhle oder die betroffene Körperöffnung eingesetzt werden.

Das textile Flächengebilde kann zugeschnitten sein oder alternativ als Endlosvlies aufgerollt und vor Ort zugeschnitten werden. Das textile Flächengebilde wird zur Verwendung als therapeutisches Mittel aufgerollt. Insbesondere ist das textile Flächengebilde in aufgerollter Form in die Körperhöhle einsetzbar. Zum Beispiel im Fall eines entzündeten äußeren Gehörganges ist das textile Flächengebilde in aufgerollter Form in den äußeren Gehörgang eingesetzt.

In einer Ausführungsform ist das textile Flächengebilde als nanostrukturiertes Hybridvlies ausgebildet. Beispielsweise ist das Hybridvlies durch Elektrospinnen einer photoresponsiven Gas-freisetzenden Substanz und einem Polymer, beispielsweise Polylactid, gebildet. Zum Beispiel weist dieses Stoffvlies eine Kohlenstoffmonoxid-induzierte antimikrobielle Aktivität auf, die ausreicht, um in Biofilmen eingebettete Bakterien nach Photostimulation beispielsweise bei 405 nm zu reduzieren. In einer Weiterbildung kann als Vlies, insbesondere Hybridvlies, Stoff oder Flächengebildet ein Photosensibilisator mit einem vorgegebenen Absorptionsbereich in einem Wellenlängenbereich von größer gleich 400 nm zur Photostimulation vorgesehen sein. Beispielsweise kann als Photosensibilisator ein Fasermaterial, ein Vlies, ein Stoff und/oder ein Flächengebilde, das zumindest Mn₂(CO)₁₀ umfasst, vorgesehen sein. Zur Aktivierung des Photosensibilisators, insbesondere der Mn₂(CO)₁₀ -Moleküle, kann dabei als Anregungslicht, insbesondere blaues oder rotes Licht, verwendet werden.

Beispielsweise ist das Hybridvlies durch Elektrospinnen eines photoresponsiven Kohlenstoffmonoxid (CO)-freisetzenden Moleküls (englisch: (CO)-releasing molecule [CORM-1, Mn₂(CO)₁₀]) und eines Polymers, beispielsweise Polylactid, hergestellt. Das freigesetzte Kohlenstoffmonoxid (CO) erhöht beispielsweise eine Konzentration reaktiver Sauerstoffspezies (englisch: ROS) in den Biofilmen.

Beispielsweise ist das Hybridvlies optional zusätzlich durch Elektrospinnen eines photoresponsiven Stickstoffmonoxid (NO)-freisetzenden Moleküls und eines Polymers hergestellt. Stickstoffmonoxid ist beispielsweise ein Neurotransmittergas mit stark antimikrobiellen Eigenschaften. Stickstoffmonoxid ist beispielsweise als Nitrosylkomplex in den Fasern und/oder im Flächengebilde eingebettet und durch Einwirkung von Licht freisetzbar. Zum Beispiel ist ein Anregungslicht mit einer Wellenlänge von zumindest 350 nm, beispielsweise 365 nm, zur Photostimulation und Aktivierung des (NO)-freisetzenden Moleküls ausreichend. Da die Wellenlänge nur zur Freisetzung des Stickstoffmonoxids genutzt wird, erfolgt keine Gewebeschädigung.

Beispielsweise umfasst das textile Flächengebilde eine Kombination aus (CO)-freisetzenden und (NO)-freisetzenden Substanzen.

Das textile Flächengebilde, insbesondere das Hybridvlies, weist nach längerer Exposition keine erhöhte Zytotoxizität auf eukaryotische Zellen auf. Daher kann sich ein solches (CO)- und/oder (NO)-freisetzende Hybridvlies als therapeutisches Mittel zur lokalen antimikrobiellen Therapie beispielsweise gegen Biofilm-assoziierte Körperhöhleninfektionen verwendet werden.

In einer Ausführungsform ist das textile Flächengebilde aus einem Stoffvlies mit biokompatiblen Polymeren, beispielsweise Polylactid, gebildet.

Die erfindungsgemäße Vorrichtung umfasst zumindest das zuvor beschriebene textile Flächengebilde zur Verwendung bei der Behandlung von einer entzündlichen Erkrankung in einer Körperhöhle. Das textile Flächengebilde ist dabei in die Körperhöhle einsetzbar. Die Vorrichtung umfasst weiter zumindest ein Dichtungselement zur Abdichtung einer, insbesondere nach außen hin gerichteten, Körperhöhlenöffnung der Körperhöhle und ein durch das Dichtungselement hindurchgeführten Lichtleiter, wobei mittels des Lichtleiters Licht in das textile Flächengebilde einbringbar und/oder das textile Flächengebilde mit Licht bestrahlbar ist. Dabei ist das zumindest eine Gas-freisetzende Molekül durch dieses Licht aktivierbar und setzt ein antibakterielles Gas frei.

Die Vorrichtung ist somit als eine lichtaktivierte Gas-freisetzende oder Therapeutikum-freisetzende, insbesondere CO-oder NO-freisetzende, Apparatur zur antibiotikafreien lokalen Behandlung von Otitis externa ausgebildet.

Die erfindungsgemäße Vorrichtung ist zur Verwendung bei einer Behandlung von entzündeten Körperhöhlen, beispielsweise Gehörgängen, gezielt einsetzbar.

In einer Ausführungsform ist das Dichtungselement aus Wachs gebildet. Das Dichtungselement ist beispielsweise ein Wachspfropfen. Das Dichtungselement kann auch aus einem anderen Material oder Werkstoff gebildet sein. Wachs ist dabei vergleichsweise kostengünstig und einfach herstellbar. Das Dichtungselement, beispielsweise ein Wachspfropfen, ist an Geometrien der Körperhöhlenöffnung flexibel anpassbar. Das Dichtungselement ist ausgebildet, die Körperhöhle gasdicht zu verschließen.

Die Vorrichtung kann beispielsweise zwei oder mehrere, voneinander beabstandete Dichtungselemente umfassen, die einen gasdicht zu verschließendem Bereich der Körperhöhle abdichten, in welchem das textile Flächengebilde angeordnet ist. Im Fall eines entzündeten Gehörganges ist eine Seite des äußeren Gehörganges bereits durch ein Trommelfell verschlossen. Das Dichtungselement ist im Bereich einer Öffnung des Gehörganges anordenbar oder angeordnet. Das textile Flächengebilde ist von außen nach innen gesehen nach dem äußeren Dichtungselement in der inneren Körperhöhle, zum Beispiel in dem äußeren Gehörgang, angeordnet. Mit anderen Worten: Das Dichtungselement dichtet eine äußere Öffnung des äußeren Gehörganges ab und das textile Flächengebilde ist zwischen der Öffnung des Gehörganges, d. h. dem Dichtungselement, und dem innenliegenden Trommelfell und somit im dazwischenliegenden äußeren Gehörgang angeordnet. Durch Abgabe des antibakteriellen Gases im gasabgedichteten Bereich der Körperhöhle, zum Beispiel des äußeren Gehörgangs, ist eine kontrollierte und gleichmäßige Behandlung des Bereichs der Körperhöhle sichergestellt.

In einer Ausführungsform ist der Lichtleiter als Glasfaser ausgebildet. Der Lichtleiter ist insbesondere dünn und flexibel ausgebildet. Daher eignet sich die Verwendung von Glasfasern, die in unterschiedlichen Körperhöhlen beispielsweise mit unterschiedlichen Durchmessern einsetzbar sind. Unter Lichtleiter ist entsprechend ein Lichtwellenleiter zum Lichttransport einer Lichtquelle zu verstehen. Natürlich können auch andere geeignete, flexible Lichtleiter bzw. Lichtwellenleiter verwendet werden. Am Ende des Lichtleiters kann eine optische Einheit, zum Beispiel eine Streulinse, angeordnet sein, die das Licht verteilt, um das Flächenmaterial großflächig zu bestrahlen.

Die Vorrichtung umfasst darüber hinaus zumindest eine geeignete Lichtquelle. Die zumindest eine Lichtquelle ist mit dem Lichtleiter zur Einkopplung von Licht in den Lichtleiter verbindbar oder verbunden. Die zumindest eine Lichtquelle kann dabei beispielsweise Licht mit einer vorgebbaren oder vorgegebenen Wellenlänge und/oder zeitweise, insbesondere für einen vorgegebenen Zeitraum, in den Lichtleiter einkoppeln.

Es hat sich gezeigt, dass, wenn Licht mit einer Wellenlänge von zumindest 400 mm und/oder für einen Zeitraum von einigen Sekunden bis zu einer Minute oder zehn Minuten, insbesondere für einen Zeitraum von 10 Sekunden oder fünf Minuten, von der Lichtquelle in den Lichtleiter eingekoppelt wird, das Molekül des textilen Flächengebildes am besten das antibakterielle Gas freisetzt.

Der Lichtleiter ist beispielsweise mit dem textilen Flächengebilde und/oder dem Dichtungselement vormontiert. Alternativ ist der Lichtleiter nach Anordnung des textilen Flächengebildes und des Dichtungselements in der Körperhöhle, beispielsweise dem Gehörgang, insbesondere dem äußeren Gehörgang, nachträglich einsetzbar. Der Lichtleiter ist durch das in der Körperhöhle angeordnete Dichtungselement, beispielsweise den Wachspfropfen, hindurchführbar, ohne dass ein Gasleck auftritt. Anschließend ist der Lichtleiter mit einer Lichtquelle koppelbar. Auch kann der Lichtleiter bereits mit einer Lichtquelle gekoppelt sein.

In einer Ausführungsform setzt die Gas-freisetzende Substanz bei Aktivierung ein antibakteriell wirkendes Gas in therapeutischen Konzentrationen frei. Die Verwendung von in höheren Konzentrationen toxischen Gasen zur Abtötung von Bakterien und Desinfektion ist bekannt.

In einer Ausführungsform setzt die Gas-freisetzende Substanz bei Aktivierung Kohlenstoffmonoxid frei. Kohlenstoffmonoxid (Kohlenmonoxid, CO) eignet sich zur Eradikation entstandener Biofilme und Bakterien. Die Vorrichtung ist vorgesehen, eine Patientenversorgung erheblich zu verbessern.

In einer Ausführungsform setzt die Gas-freisetzende Substanz bei Aktivierung Kohlenstoffmonoxid und/oder Stickstoffmonoxid frei.

Kohlenstoffmonoxid (CO) ist ein Toxin, das die Schlüsselenzyme der essentiellen Elektronentransportkette, wie Cytochrome oder Cytochrom c-Oxidase, hemmt und so zum Absterben von Bakterien führt.

In einer Ausführungsform umfasst die Vorrichtung zumindest eine Lichtquelle, die mit dem Lichtleiter koppelbar oder gekoppelt ist, wobei der Lichtleiter Licht der Lichtquelle an das textile Flächengebilde zur Aktivierung des Gas-freisetzenden Moleküls weiterleitet. Am Ende des Lichtleiters kann eine optische Einheit, zum Beispiel eine Streulinse, angeordnet sein, die das Licht verteilt, um das Flächenmaterial großflächig zu bestrahlen.

In einer Ausführungsform leitet der Lichtleiter Licht der Lichtquelle in einer Wellenlänge von 400 bis 800 nm, beispielsweise von 405 bis 600 nm zur Aktivierung der Gas-freisetzenden Substanz an das textile Flächengebilde weiter und koppelt es in dieses ein oder bestrahlt es mit diesem Licht. Die Wellenlänge und der Zeitraum sind beispielsweise vorprogrammierbar und/oder einstellbar und beträgt weniger als 10 min.

Ein Verfahren zur Herstellung der Vorrichtung zur Verwendung bei der Behandlung von Bakterien in einer Körperhöhle umfasst folgende Schritte:
- Einlegen eines textilen Flächengebildes in die Körperhöhle mit einer nach außen hin offenen Körperhöhlenöffnung, wobei das textile Flächengebilde zumindest eine Gas-freisetzendes Substanz umfasst,
- Einlegen eines Dichtungselements im Bereich der Körperhöhlenöffnung und Abdichten der Körperhöhlenöffnung und
- Hindurchführen eines Lichtleiters durch das Dichtungselement,
- Einkopplung von Licht in den Lichtleiter und Transport des Lichts durch das Dichtungselement hindurch und Einbringen des Lichts auf oder in das textile Flächengebilde. Durch das Einbringen von Licht auf oder in das textile Flächengebilde wird das enthaltende Molekül oder die Substanz aktiviert und antibakterielle Gase in der Körperhöhle freigesetzt.

Im Fall eines zu behandelnden Gehörganges, beispielsweise bei Otitis externa wird die Vorrichtung wie folgt bei der Behandlung der Otits externa verwendet:
Der äußere Gehörgang ist auf der einen Seite durch das Trommelfell verschlossen. Das textile Flächengebilde, insbesondere ein Vliesgebilde, wird durch die Körperhöhlenöffnung, d. h. die äußere Öffnung des äußeren Gehörganges, in einen hinteren Bereich der Körperhöhle, d. h. in den äußeren Gehörgang, eingeschoben. Zuvor wird das textile Flächengebilde beispielsweise aufgerollt. Zur gasdichten Abdichtung des zu behandelnden äußeren Gehörganges wird das Dichtungselement in die Körperhöhlenöffnung, insbesondere der äußeren Gehörgangöffnung, eingesetzt, so dass der äußere Gehörgang gasdicht verschlossen ist. Ein Ende des Lichtleiters, insbesondere die Glasfaser, wird durch das beispielsweise als Wachspfropfen ausgebildete Dichtungselement hindurchgeführt, beispielsweise geschoben. Das hindurchgeführte Ende des Lichtleiters wird im inneren Bereich des äußeren Gehörgangs zur Bestrahlung des textilen Flächengebildes angeordnet. Anschließend wird das andere, insbesondere äußere, freie Ende des Lichtleiters mit einer Lichtquelle verbunden. Diese Lichtquelle gibt Licht der Wellenlänge von beispielsweise 300 bis 800 nm, beispielsweise 365 nm bis 480 nm, aus. Der Lichtleiter leitet dieses Licht weiter und bestrahlt das mit einer Gas-freisetzenden Substanz versehene textile Flächengebilde im äußeren Gehörgang zur Aktivierung der Substanz und Freisetzung des antibakteriellen Gases. Durch Aktivierung der Substanz mittels des eingebrachten Lichts wird stoßartig Gas, beispielsweise Kohlenstoffmonoxid oder Stickstoffoxid, freigesetzt. Dieses freisetzende Kohlenstoffmonoxid-Gas oder Stickstoff-Gas verteilt sich aufgrund seiner Gaseigenschaft gleichmäßig im abgedichteten und verschlossenen äußeren Gehörgang. Das Dichtungselement verbleibt für eine vorbestimmte Zeit in situ. Diese Behandlung kann natürlich des Öfteren wiederholt werden.

In einer Weiterbildung ist die erfindungsgemäße Vorrichtung in Form eines Ohrpassstücks oder Ohrstöpsels ausgebildet. Die Vorrichtung umfasst beispielsweise ein Gehäuse, umfassend die zuvor beschriebenen Komponenten.

Zum Beispiel umfasst das Gehäuse ein erstes Gehäuseteil, welches außerhalb der Körperhöhlenöffnung anordenbar bzw. angeordnet ist. Das Gehäuse umfasst beispielsweise ein zweites Gehäuseteil, welches durch die Körperhöhlenöffnung in die Körperhöhle einführbar bzw. eingeführt ist. Die Vorrichtung umfasst beispielsweise eine Batterie, eine Lichtquelle, beispielswiese eine Anzahl von Leuchtdioden, zur Lichtaktivierung der Gas-freisetzenden Substanz und eine Elektronik zum zeitgesteuerten Betrieb einer Lichtquelle, einen Lichtleiter sowie ein integriertes textiles Flächengebilde mit einer Anzahl von Gas-freisetzenden Molekülen oder einer Gas-freisetzenden Substanz. Eine Verwendung von einzelnen Komponenten kann dabei vermieden werden.

Eine weitere Ausführungsform sieht vor, dass die Gasfreisetzung getriggert werden kann. Insbesondere kann eine Konzentration des freisetzenden Gases gesteuert werden. Hierzu kann die Vorrichtung zusätzlich eine Steuereinheit, insbesondere zur Steuerung der Lichtquelle, umfassen, welche eine intervallartige Aktivierung der Lichtquelle und damit eine intervallartige Bestrahlung und Freisetzung des Gases ermöglicht. Hingegen kann bei einer Dauerbeleuchtung das gesamte Gas auf einmal freigesetzt werden. Eine intervallartige, eine prolongierte oder eine dauerhafte Bestrahlung durch entsprechende Steuerung der Lichtquelle mittels der Steuereinheit ermöglicht, dass das antibakterielle Gas über einen spezifischen Zeitraum in therapeutisch relevanten Dosierungen freigesetzt wird.

Ein Ohrpassstück, Ohreinsatz oder ein Ohrstöpsel ist ein unkompliziert anzuwendendes Produkt. Die Batterie, Elektronik sowie Lichtquelle sind beispielsweise im ersten Gehäuseteil angeordnet. Das erste Gehäuseteil ist beispielsweise aus einem härteren Material als das zweite Gehäuseteil ausgebildet. Das zweite Gehäuseteil ist beispielsweise aus Wachs, Schaumstoff und/oder Silikon gebildet. Das zweite Gehäuseteil ist beispielsweise zur Abdichtung der Körperhöhlenöffnung ausgebildet. Das zweite Gehäuseteil umfasst beispielsweise das Dichtungselement, einen Abschnitt des Lichtleiters und das textile Flächengebilde. Das zweite Gehäuseteil ist ausgebildet, die Körperhöhle gasdicht zu verschließen und das flexible Flächengebilde in der Körperhöhle zu halten.

Ausführungsbeispiele der Erfindung werden im Folgenden anhand von Zeichnungen näher erläutert.

Darin zeigen:
- Figuren 1 und 2: schematisch verschiedene Ausführungsformen eines textilen Flächengebildes als therapeutisches Mittel,
- Figur 3: schematisch eine erfindungsgemäße Vorrichtung zur Verwendung bei einer Behandlung von Entzündungen in einer Körperhöhle,
- Figur 4: schematisch ein erfindungsgemäßes Verfahren zur Herstellung einer Vorrichtung bei einer Behandlung von Entzündungen in einer Körperhöhle,
- Figur 5: schematisch eine Ausführungsform einer erfindungsgemäßen Vorrichtung zur Verwendung bei einer Behandlung von Entzündungen in einer Körperhöhle, und
- Figur 6: schematisch eine weitere Ausführungsform einer erfindungsgemäßen Vorrichtung zur Verwendung bei einer Behandlung von Entzündungen in einer Körperhöhle.

Einander entsprechende Teile sind in allen Figuren mit den gleichen Bezugszeichen versehen.

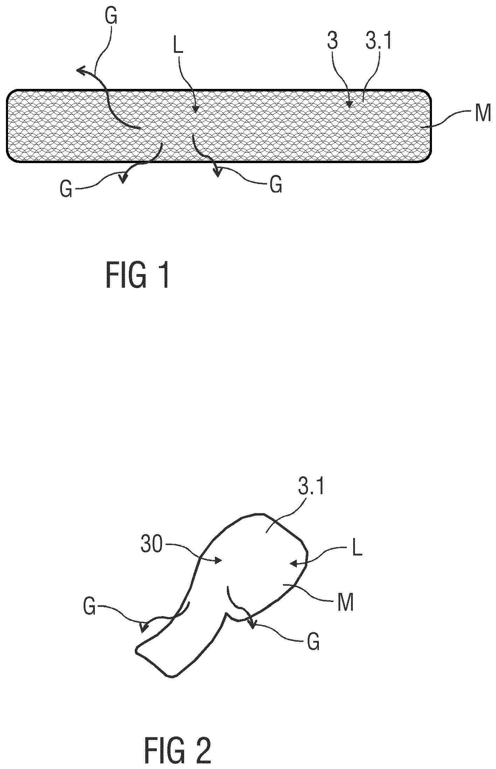

**Figuren 1 und 2** zeigen jeweils eine Ausführungsform eines textilen Flächengebildes 3 bzw. 30.

Das textile Flächengebilde 3 bzw. 30 ist als ein Vlies 3.1, insbesondere ein Stoffvlies ausgebildet. Das Vlies 3.1 kann aus losen Fasern oder als Gewebe ausgebildet sein.

In Figur 1 ist das textile Flächengebilde 1 als Endlosmaterial dargestellt, welches zur Verwendung als therapeutisches Mittel zugeschnitten werden kann und beispielsweise als flächiges Auflagemittel oder in aufgerollter Form in Art eines Pfropfens einsetzbar ist.

In Figur 2 ist das textile Flächengebilde 30 zugeschnitten und in aufgerollter Form gezeigt, in welcher das textile Flächengebilde 30 als therapeutisches Mittel verwendet wird.

Im Nachfolgenden wird das textile Flächengebilde 3, 30 anhand des textilen Flächengebildes 3 beschrieben. Die Ausführungen zum textilen Flächengebilde 3 bis auf die Form gelten analog für das textile Flächengebilde 30.

Das textile Flächengebilde 3 ist mit zumindest einer Gas-freisetzenden Substanz M versehen, welches bei Aktivierung durch aufgebrachtes oder eingekoppeltes Licht L ein antibakterielles Gas G freisetzt. Insbesondere setzt die Gas-freisetzende Substanz M bei Aktivierung ein toxisches Gas G, wie zum Beispiel Kohlenstoffmonoxid, frei.

Hierzu ist die Gas-freisetzende Substanz M oder das Gas-freisetzende Molekül beispielsweise ein Carbonylkomplex eines Übergangsmetalls. Insbesondere ist die Gas-freisetzende Substanz M oder das Gas-freisetzende Molekül zumindest Mn₂(CO)₁₀.

Darüber hinaus ist das textile Flächengebilde 3 als ein Stoffvlies 3.1 ausgebildet. Beispielsweise ist das textile Flächengebilde 3 als ein nanostrukturiertes Hybridvlies ausgebildet. Beispielsweise ist das Hybridvlies durch Elektrospinnen einer photoresponsiven Gas-freisetzenden Substanz M und einem Polymer, beispielsweise Polylactid, gebildet. Zum Beispiel weist dieses Stoffvlies 3.1 eine Kohlenstoffmonoxid-induzierte antimikrobielle Aktivität auf, die ausreicht, um in Biofilmen eingebettete Bakterien nach Photostimulation beispielsweise bei 405 nm zu reduzieren. Beispielsweise ist das Hybridvlies durch Elektrospinnen einer photoresponsiven Kohlenstoffmonoxid (CO)-freisetzenden Substanz M (englisch: (CO)-releasing molecule [CORM-1, Mn2(CO)10]) und eines Polymers, beispielsweise Polylactid, hergestellt. Das freigesetzte Kohlenstoffmonoxid (CO) erhöht beispielsweise die Konzentration reaktiver Sauerstoffspezies (englisch: ROS) in den Biofilmen. Das textile Flächengebilde 3.1, insbesondere das Hybridvlies, weist nach längerer Exposition eine erhöhte Zytotoxizität auf eukaryotische Zellen auf. Daher kann sich ein solches (CO)-freisetzende Hybridvlies als therapeutisches Mittel zur lokalen antimikrobiellen Therapie beispielsweise gegen Biofilm-assoziierte Körperhöhleninfektionen verwendet werden. Insbesondere kann das textile Flächengebilde 3 als therapeutisches Mittel bei Behandlung von entzündlichen Erkrankungen in einer Körperhöhle, wobei die entzündliche Erkrankung beispielsweise eine Ohrenentzündung, eine Achselentzündung, eine Hautentzündung in einer Hautfalte oder eine Otitis externa ist.

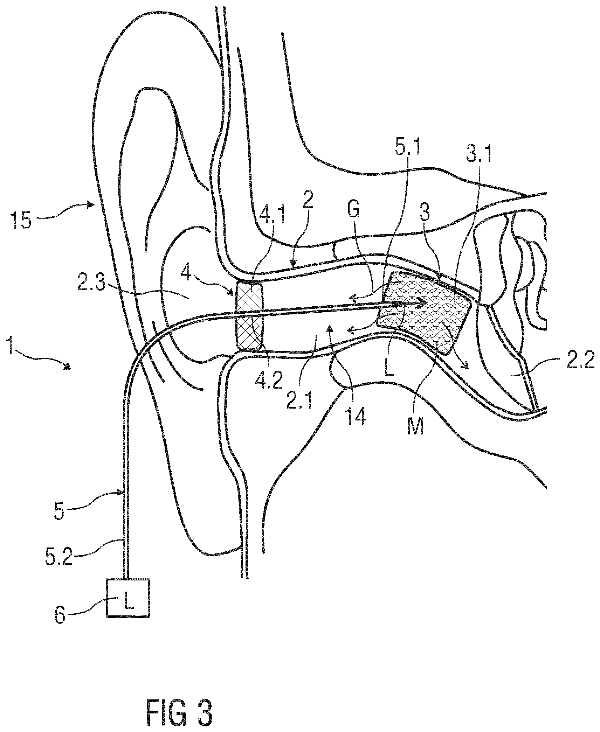

**Figur 3** zeigt eine Vorrichtung 1 zur Verwendung bei einer Behandlung einer entzündlichen Erkrankung in einer Körperhöhle 2. Die Vorrichtung 1 ist eine lichtaktivierte, Gas-freisetzende Apparatur zur antibiotikafreien lokalen Therapie von Körperhöhlen 2 mit Infektionen.

Die dargestellte Vorrichtung 1 ist beispielsweise zur Verwendung bei einer Behandlung einer durch Bakterien hervorgerufenen Infektion und/oder Entzündung eines äußeren Gehörganges 2.1 vorgesehen. Die Vorrichtung 1 ist im dargestellten Ausführungsbeispiel teilweise im Gehörgang 2.1 verbaut und zur Therapie einsatzbereit. Die Vorrichtung 1 ist beispielsweise zur Behandlung bzw. Therapie einer so genannten Otitis externa vorgesehen.

Die Vorrichtung 1 umfasst das textile Flächengebilde 3 zum Einlegen in die Körperhöhle 2. Das textile Flächengebilde 3 kann als Einlage oder in aufgerollter Form in den Gehörgang 2.1 angeordnet werden.

Die Körperhöhle 2 ist beispielsweise ein Gehörgang 2.1, insbesondere der äußere Gehörgang. Das textile Flächengebilde 3 ist beispielsweise ein Stoffvlies 3.1. Das textile Flächengebilde 3 ist in einem inneren Bereich der Körperhöhle 2 angeordnet. Beispielsweise ist das textile Flächengebilde 3 innerhalb des Gehörgang 2.1 vor dem Trommelfell 2.2 hin, d. h. proximal, angeordnet.

Des Weiteren umfasst die Vorrichtung 1 ein Dichtungselement 4 zur Abdichtung einer Körperhöhlenöffnung 2.3 des Gehörgangs 2.1. Die Körperhöhlenöffnung 2.3 ist beispielsweise eine distale Öffnung des Gehörganges 2.1. Das Dichtungselement 4 ist beispielsweise aus Wachs gebildet. Das Dichtungselement 4 ist beispielsweise ein Pfropfen 4.1. Das Dichtungselement 4 ist vollständig in der Körperhöhlenöffnung 2.3 angeordnet und dichtet die Körperhöhle 2, insbesondere den Gehörgang 2.1, vollständig nach außen ab.

Weiterhin umfasst die Vorrichtung 1 einen Lichtleiter 5 zum Lichttransport. Der Lichtleiter 5 ist flexibel und beispielsweise eine Glasfaser. Der Lichtleiter 5 ist durch das Dichtungselement 4 hindurchgeführt. Ein durch das Dichtungselement 4 hindurchgeführtes Ende 5.1 des Lichtleiters 5 ist in der Körperhöhle 2 angeordnet. Dieses Ende 5.1 ragt in Richtung des Trommelfells 2.2 in den Gehörgang 2.1 hinein. Insbesondere ist das Ende 5.1 des Lichtleiters 5 in Nähe des textilen Flächengebildes 3 angeordnet. Am Ende 5.1 des Lichtleiters 5 kann darüber hinaus eine nicht näher dargestellte optische Einheit, zum Beispiel eine Streulinse, angeordnet sein, die das Licht verteilt, um das Flächenmaterial, insbesondere das textile Flächengebilde 3, großflächig zu bestrahlen.

Das andere Ende 5.2 des Lichtleiters 5 ist mit einer Lichtquelle 6 gekoppelt. Das "innere" Ende 5.1 des Lichtleiters 5 kann in einfacher Weise durch das Dichtungselement 4 in den hinteren Bereich des Gehörganges 2.1 eingeschoben werden. Da das Dichtungselement 4 aus Wachs gebildet ist, verformt sich das Dichtungselement 4 beim Eindringen und Durchstechen des Lichtleiters 5, wodurch eine Öffnung 4.2, insbesondere ohne Entstehung eines Gaslecks, im Dichtungselement 4 ausbildbar bzw. ausgebildet ist.

Der Lichtleiter 5 ist vorgesehen, von der Lichtquelle 6 eingebrachtes Licht L an das textile Flächengebilde 3 aufzubringen oder in dieses einzukoppeln. Das textile Flächengebilde 3 umfasst eine Anzahl oder Mehrzahl von Gas-freisetzenden Substanzen M. Beispielsweise ist zumindest eine Gas-freisetzende Substanz M in dem Flächengebilde 3 eingebettet. Das mit der Gas-freisetzenden Substanz M versehene Flächengebilde 3 ist bei Einbringung oder bei Bestrahlung von Licht L derart aktivierbar, dass die Substanz M ein Gas G freisetzt. Insbesondere ist die Substanz M durch das durch den Lichtleiter 5 eingebrachte Licht L aktivierbar, dass ein antibakterielles Gas G zur Eradikation von Bakterien freigesetzt wird. Das vom Flächengebilde 3 abgegebene antibakterielle Gas G verteilt sich gleichmäßig innerhalb der abgedichteten Körperhöhle 2. Im dargestellten Ausführungsbeispiel verteilt sich das antibakterielle Gas G gleichmäßig innerhalb des abgedichteten Gehörganges 2.1 zwischen Trommelfell 2.2 und Dichtungselement 4.

Das antibakterielle Gas G ist beispielsweise Kohlenstoffmonoxid (CO). Die Substanz M ist eine CO-freisetzende Substanz.

Beispielsweise ist die CO-freisetzende Substanz M durch ein vom Lichtleiter 5 eingebrachtes Licht L mit einer Wellenlänge von 405 nm und/oder einer Zeitdauer von einigen Sekunden, zum Beispiel 10 Sekunden, bis zu einer Minute oder zehn Minuten, insbesondere von fünf Minuten, aktivierbar.

In einer nicht näher dargestellten Ausführungsform umfasst die Vorrichtung 1 zwei Dichtungselemente 4 zur Abdichtung des Körperhöhlenbereichs. Das mit Gas-freisetzenden Molekülen oder der Gas-freisetzenden Substanz M versehene textile Flächengebilde 3 kann beispielsweise dann zwischen den beiden Dichtungselementen 4 angeordnet sein. Ein Dichtungselement 4 ist dabei proximal in der Körperhöhle 2 angeordnet und das andere Dichtungselement 4 distal in Bezug auf die Körperhöhle 2 angeordnet. Der mit der Lichtquelle 6 gekoppelte Lichtleiter 5 ist durch das distal angeordnete Dichtungselement 4 hindurchgeführt. Dadurch kann ein beliebiger, abgedichteter und geschlossener Körperhöhlenbereich therapiert bzw. behandelt werden. Auch kann das textile Flächengebilde 3 nach den mehreren Dichtungselementen 4 und vor dem Trommelfell 2.2 angeordnet sein, um eine sichere Abdichtung der Körperhöhle 2 zu ermöglichen.

Die Vorrichtung 1 ist zum Einsetzen in ein Innenohr 14 und/oder ein Außenohr 15 eines Kopfes vorgesehen. Im Ausführungsbeispiel nach Figuren 1 bis 3 sind zumindest das textile Flächengebilde 3 und das Dichtungselement 4 im Innenohr 14 eingesetzt. Die Lichtquelle 6 kann am Außenohr 15 oder zu diesem beabstandet angeordnet sein.

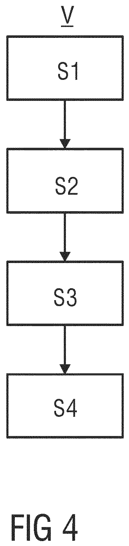

**Figur 4** zeigt ein Verfahren V zur Herstellung der Vorrichtung 1 zur Verwendung bei einer Behandlung von Infektionen und Entzündungen in einer Körperhöhle 2.

Das Verfahren V umfasst folgende Schritte:
Verfahrensschritt S1: Einlegen des textilen Flächengebildes 3 oder 30 in die Körperhöhle 2 mit einer nach außen hin offenen Körperhöhlenöffnung 2.3, wobei das textile Flächengebilde 3 oder 30 mit zumindest einer Gas-freisetzenden Substanz M zuvor versehen wurde oder bereits versehen ist.
Verfahrensschritt S2: Einlegen eines Dichtungselements 4 im Bereich der Körperhöhlenöffnung 2.3 und Abdichten der Körperhöhlenöffnung 2.3.
Verfahrensschritt S3: Hindurchführen des Lichtleiters 5 durch das Dichtungselement 4.
Verfahrensschritt S4: Einkopplung von Licht L der Lichtquelle 6 in den Lichtleiter 5 und Transport des Lichts L durch das Dichtungselement 4 hindurch und Einbringen des Lichts L auf oder in das textile Flächengebilde 3 zur Aktivierung der in dem textilen Flächengebilde 3 vorgesehenen Substanz M und zur Freisetzung des antibakterielles Gases G durch die aktivierte Substanz M.

Im Fall eines zu behandelnden Gehörganges 2.1, beispielsweise bei Otitis externa:
Der äußere Gehörgang 2.1 ist auf der einen Seite durch das Trommelfell 2.2 verschlossen. Das textile Flächengebilde 3 wird durch die Körperhöhlenöffnung 2.3, d. h. durch die Öffnung des Gehörganges 2.1, in den hinteren Bereich der Körperhöhle 2, d. h. des Gehörganges 2.1, eingeschoben. Zuvor wird das textile Flächengebilde 3 beispielsweise aufgerollt. Das textile Flächengebilde 3 ist vorbehandelt und mit einer Anzahl oder Mehrzahl von Gas-freisetzenden Molekülen oder einer Gas-freisetzenden Substanz M versehen. Zur gasdichten Abdichtung des zu behandelnden äußeren Gehörganges 2.1 wird das Dichtungselement 4 in die Körperhöhlenöffnung 2.3, insbesondere der Gehörgangöffnung, eingesetzt und verschlossen. Der Lichtleiter 5, insbesondere die Glasfaser, wird durch das beispielsweise als Pfropfen 4.1, insbesondere Wachspfropfen, ausgebildete Dichtungselement 4 hindurchgeführt, beispielsweise geschoben. Das innere Ende 5.1 des Lichtleiters 5 wird im hinteren Bereich der Körperhöhle 2 zur Bestrahlung des textilen Flächengebildes 3 angeordnet. Anschließend wird der Lichtleiter 5 an die Lichtquelle 6 angeschlossen. Diese Lichtquelle 6 gibt Licht L der Wellenlänge von beispielsweise 405 nm aus. Der Lichtleiter 5 leitet dieses Licht L weiter und bestrahlt das mit der Gas-freisetzenden Substanz M oder mehreren Molekülen versehene Flächengebilde 3 zur Aktivierung und Freisetzung von antibakteriell wirkenden Gasen G. Durch Aktivierung der Substanz M oder der Molekülemittels des eingebrachten Lichts L wird stoßartig Gas G, beispielsweise Kohlenstoffmonoxid, freigesetzt, das sich aufgrund seiner Gaseigenschaft gleichmäßig im abgedichteten und verschlossenen äußeren Gehörgang 2.1 verteilt. Das Dichtungselement 4 verbleibt für eine vorbestimmte Zeit in situ. Diese Behandlung kann natürlich des Öfteren wiederholt werden.

Im Fall eines nicht näher dargestellten, anderen zu behandelnden Körperhöhlenbereichs:
Zur gezielten Abdichtung eines bestimmten Körperhöhlenbereichs wird zunächst ein erstes Dichtungselement 4 durch eine Körperhöhlenöffnung 2.3 in einen proximalen Bereich einer Körperhöhle 2 zur gasdichten Abdichtung eingeschoben. Anschließend wird ein vorbehandeltes textiles Flächengebilde 3 durch die Körperhöhlenöffnung 2.3 eingeschoben. Zuvor wird das textile Flächengebilde 3 beispielsweise aufgerollt. Das textile Flächengebilde 3 ist vorbehandelt und mit einer Anzahl oder Mehrzahl von Gas-freisetzenden Substanzen M versehen. Zur weiteren gasdichten Abdichtung des zu behandelnden Körperhöhlenbereichs wird ein zweites Dichtungselement 4 in die Körperhöhlenöffnung 2.3 eingesetzt und verschlossen. Der Lichtleiter 5, insbesondere die Glasfaser, wird durch das zweite Dichtungselement 4 hindurchgeführt, beispielsweise geschoben. Ein Ende 5.1 des Lichtleiters 5 wird also zwischen den beiden Dichtungselementen 4 zur Bestrahlung des textilen Flächengebildes 3 angeordnet. Anschließend wird der Lichtleiter 5 an die Lichtquelle 6 angeschlossen. Diese Lichtquelle 6 gibt Licht L der Wellenlänge von beispielsweise 405 nm aus. Der Lichtleiter 5 leitet dieses Licht L weiter und bestrahlt das mit der Gas-freisetzenden Substanz M oder mehreren Molekülen versehene Flächengebilde 3 zur Aktivierung und Freisetzung von antibakteriell wirkenden Gasen G.

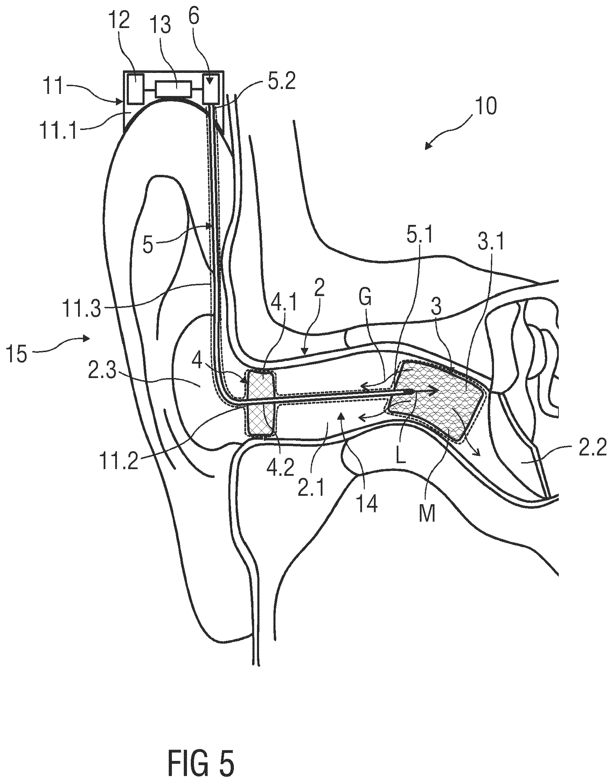

**Figur 5** zeigt schematisch eine weitere Ausführungsform einer erfindungsgemäßen Vorrichtung 10 zur Verwendung bei einer Behandlung einer entzündlichen Erkrankung in einer Körperhöhle 2. Die Vorrichtung 10 ist eine lichtaktivierte, Gas-freisetzende Apparatur zur antibiotikafreien lokalen Therapie von Körperhöhlen 2 mit Infektionen.

Die dargestellte Vorrichtung 10 ist beispielsweise in Form eines Ohrpassstücks, ähnlich eines Hörgeräts, ausgebildet und zur Verwendung bei einer Behandlung einer durch Bakterien hervorgerufenen Infektion und/oder Entzündung eines äußeren Gehörganges 2.1 vorgesehen.

Die Vorrichtung 10 ist im dargestellten Ausführungsbeispiel teilweise im Gehörgang 2.1 angeordnet und zur Therapie einsatzbereit. Die Vorrichtung 10 ist beispielsweise ein von einer Person tragbares Gerät. Die Vorrichtung 10 ist beispielsweise zur Behandlung bzw. Therapie einer so genannten Otitis externa vorgesehen. Beispielsweise ist die Vorrichtung 10 teilweise im Gehörgang 2.1 angeordnet und außerhalb des Gehörgangs 2.1 am Kopf und/oder hinter dem Ohr befestigt.

Die Vorrichtung 10 umfasst die zuvor beschriebenen Komponenten der Vorrichtung 1 und ist im Wesentlichen nach dem Ausführungsbeispiel gemäß Figur 3 ausgebildet. Zusätzlich umfasst die Vorrichtung 10 eine Batterie 12 und eine Elektronik 13 zum zeitgesteuerten Betrieb der Lichtquelle 6 zur Lichtaktivierung der Gas-freisetzenden Substanz M.

Im dargestellten Ausführungsbeispiel umfasst die Vorrichtung 10 zusätzlich ein Gehäuse 11 zur Einhausung der zuvor genannten Komponenten. Dadurch ist die Vorrichtung 10 als unkompliziert anzuwendendes Produkt bzw. bereits vormontiertes Gerät verwendbar.

Die Vorrichtung 10 ist zum Einsetzen in das Innenohr 14 und/oder das Außenohr 15 eines Kopfes vorgesehen. Im Ausführungsbeispiel nach Figur 5 sind zumindest das textile Flächengebilde 3 und das Dichtungselement 4 im Innenohr 14 eingesetzt. Die Lichtquelle 6, die Batterie 12 und die Elektronik 13 können am Außenohr 15 oder zu diesem beabstandet angeordnet sein.

Das Gehäuse 11 weist ein erstes Gehäuseteil 11.1, beispielsweise einen Gehäuseabschnitt, auf. Im ersten Gehäuseteil 11.1 sind beispielsweise die Lichtquelle 6, die Batterie 12 und die Elektronik 13 angeordnet. Das erste Gehäuseteil 11.1 ist außerhalb des Gehörganges 2.1 anzuordnen bzw. angeordnet.

Das Gehäuse 11 weist ein zweites Gehäuseteil 11.2, beispielsweise einen Gehäuseabschnitt, auf. Im zweiten Gehäuseteil 11.2 sind das Dichtungselement 4.2, ein Teil des Lichtleiters 5 sowie das textile Flächengebilde 3 angeordnet. Das zweite Gehäuseteil 11.2 ist insbesondere innerhalb des Gehörganges 2.1 anzuordnen bzw. angeordnet. Das zweite Gehäuseteil 11.2 ist beispielsweise aus weichem Silikon gebildet.

Im Bereich des textilen Flächengebildes 3 ist das zweite Gehäuseteil 11.2 für die Substanz M durchlässig ausgebildet. Das zweite Gehäuseteil 11.2 weist im Bereich des textilen Flächengebildes 3 beispielsweise eine Mehrzahl von nicht näher dargestellten Durchgangsöffnungen auf. Das erste Gehäuseteil 11.1 kann beispielsweise in Form eines Hörgeräts ausgebildet sein.

Zur Führung des Lichtleiters 5 von der Lichtquelle 6 zum textilen Flächengebilde 3 weist das Gehäuse 11 ein Verbindungsstück 11.3 auf. Das Verbindungsstück 11.3 ist beispielsweise rohrförmig ausgebildet. Das Verbindungsstück 11.3 ist flexibel ausgebildet. In einem Hohlraum des Verbindungsstücks 11.3 ist der Lichtleiter 5 angeordnet und hindurchgeführt. Das Verbindungsstück 11.3 ist beispielsweise aus weichem Silikon gebildet. Das Verbindungsstück 11.3 koppelt beide Gehäuseteile 11.1, 11.2.

Das zweite Gehäuseteil 11.2 ist beispielsweise in Form eines Ohrstöpsels ausgebildet. Das zweite Gehäuseteil 11.2 ist beispielsweise reversibel flexibel oder deformierbar ausgebildet. Dadurch ist das zweite Gehäuseteil 11.2 in einfacher Weise in die Körperhöhle 2 einführbar bzw. einschiebbar. Das erste Gehäuseteil 11.1 ist beispielsweise aus einem härteren Material gebildet und an außerhalb der Körperhöhle 2 anordenbar und fixierbar.

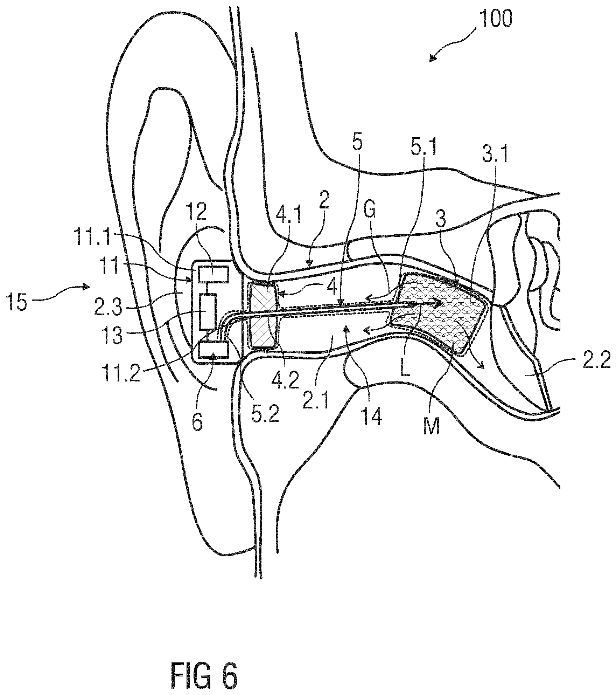

**Figur 6** zeigt schematisch eine weitere alternative Ausführungsform einer Vorrichtung 100 zur Verwendung bei einer Behandlung einer entzündlichen Erkrankung in einer Körperhöhle 2. Die Vorrichtung 100 ist eine lichtaktivierte, Gas-freisetzende Apparatur zur antibiotikafreien lokalen Therapie von Körperhöhlen 2 mit Infektionen.

Die dargestellte Vorrichtung 100 ist beispielsweise in Form eines Ohrpassstücks, ähnlich eines Hörgeräts, ausgebildet und zur Verwendung bei einer Behandlung einer durch Bakterien hervorgerufenen Infektion und/oder Entzündung eines äußeren Gehörganges 2.1 vorgesehen.

Die Vorrichtung 100 ist im dargestellten Ausführungsbeispiel im Gehörgang 2.1 angeordnet und zur Therapie einsatzbereit. Die Vorrichtung 100 ist beispielsweise ein von einer Person tragbares Gerät. Die Vorrichtung 100 ist beispielsweise zur Behandlung bzw. Therapie einer so genannten Otitis externa vorgesehen.

Die Vorrichtung 100 umfasst die zuvor beschriebenen Komponenten und ist im Wesentlichen nach dem Ausführungsbeispiel gemäß Figur 5 ausgebildet. Dabei sind die Batterie 12, die Elektronik 13 und die Lichtquelle 6 als eine integrierte Baueinheit ausgebildet und teilweise im Bereich des Innenohres 14 angeordnet. Beispielsweise sind die Batterie 12, die Elektronik 13 und die Lichtquelle 6 zur Lichtaktivierung der Gas-freisetzenden Substanz M am Dichtelement 4, insbesondere auf dessen nach außen, vom Innenohr 14 weg gerichteten Seite, angeordnet.

Im dargestellten Ausführungsbeispiel können die Batterie 12, die Elektronik 13 und die Lichtquelle 6 von dem Gehäuse 11 gekapselt sein. Eine solche Vorrichtung 100 ist im Wesentlichen zum Einsetzen im Innenohr 14 ausgebildet.

Eine weitere Ausführungsform sieht vor, dass die Gasfreisetzung getriggert werden kann. Insbesondere kann eine Konzentration des freisetzenden Gases G gesteuert werden. Hierzu kann die Vorrichtung 1, 10 oder 100 zusätzlich eine Steuereinheit, insbesondere zur Steuerung der Lichtquelle 6, umfassen, welche eine intervallartige Aktivierung der Lichtquelle 6 und damit eine intervallartige Bestrahlung und Freisetzung des Gases G ermöglicht. Die Steuereinheit kann dabei Teil der Elektronik 13 oder separat ausgebildet sein. Hingegen kann bei einer Dauerbeleuchtung das gesamte Gas G auf einmal freigesetzt werden. Dies ist häufig unerwünscht, da es zu einer Überdosierung führen kann. Eine intervallartige Bestrahlung durch entsprechende Steuerung der Lichtquelle 6 mittels der Steuereinheit ermöglicht, dass das antibakterielle Gas G über einen langen Zeitraum in therapeutisch relevanten Dosierungen freigesetzt wird.

### BEZUGSZEICHENLISTE

- 1, 100: Vorrichtung
- 2: Körperhöhle
- 2.1: Gehörgang
- 2.2: Trommelfell
- 2.3: Körperhöhlenöffnung
- 3, 30: Textiles Flächengebilde
- 3.1: Stoffvlies
- 4: Dichtungselement
- 4.1: Pfropfen
- 4.2: Öffnung
- 5: Lichtleiter
- 5.1, 5.2: Ende
- 6: Lichtquelle
- 10: Vorrichtung
- 11: Gehäuse
- 11.1, 11.2: Gehäuseteil
- 11.3: Verbindungsstück
- 12: Batterie
- 13: Elektronik
- 14: Innenohr
- 15: Außenohr

- G: Gas
- L: Licht
- M: Substanz
- V: Verfahren
- S1 bis S4: Verfahrensschritt

## Patentansprüche

1. Vorrichtung (1, 10, 100), umfassend ein textiles Flächengebilde (3, 30) zur Verwendung als therapeutisches Mittel bei einer Behandlung einer entzündlichen Erkrankung in einer Körperhöhle (2),
wobei das textile Flächengebilde (3, 30) mit zumindest einer Gas-freisetzenden Substanz (M) versehen ist, welche bei Aktivierung durch Licht (L) ein antibakterielles Gas (G) freisetzt,
**dadurch gekennzeichnet, dass** die zu behandelnde Körperhöhle (2) ein Gehörgang ist,
wobei die Vorrichtung (1, 10, 100) weiter ein Dichtungselement (4) zur Abdichtung des Gehörgangs und einen durch das Dichtungselement (4) hindurchgeführten Lichtleiter (5) umfasst,
wobei mittels des Lichtleiters (5) Licht (L) in das textile Flächengebilde (3, 30) einbringbar und/oder das textile Flächengebilde (3, 30) mit Licht (L) bestrahlbar ist,
wobei die zumindest eine Gas-freisetzende Substanz (M) durch dieses Licht (L) aktivierbar ist und ein antibakterielles Gas (G) freisetzt.

2. Vorrichtung (1, 10, 100) nach Anspruch 1, wobei die Gas-freisetzende Substanz (M) bei Aktivierung ein toxisches Gas (G) freisetzt.

3. Vorrichtung (1, 10, 100) nach Anspruch 1 oder 2, wobei die Gas-freisetzende Substanz (M) bei Aktivierung Kohlenstoffmonoxid und/oder Stickstoffmonoxid freisetzt.

4. Vorrichtung (1, 10, 100) nach einem der vorhergehenden Ansprüche, wobei die Gas-freisetzende Substanz (M) ein Carbonylkomplex eines Übergangsmetalls oder ein Nitrosylkomplex ist.

5. Vorrichtung (1, 10, 100) nach einem der vorhergehenden Ansprüche, wobei die Gas-freisetzende Substanz (M) zumindest Mn₂(CO)₁₀ ist.

6. Vorrichtung (1, 10, 100) nach einem der vorhergehenden Ansprüche, **gekennzeichnet durch** ein Stoffvlies (3.1).

7. Vorrichtung (1, 10, 100) nach Anspruch 6, wobei das Stoffvlies (3.1) ein nanostrukturiertes Hybridvlies ist.

8. Vorrichtung (1, 10, 100) nach einem der vorhergehenden Ansprüche, wobei das Dichtungselement (4) aus Wachs gebildet ist.

9. Vorrichtung (1, 10, 100) nach einem der vorhergehenden Ansprüche, weiter umfassend zumindest eine Lichtquelle (6), die mit dem Lichtleiter (5) zur Einkopplung von Licht (L) in den Lichtleiter (5) verbindbar oder verbunden ist.

10. Vorrichtung (1, 10, 100) nach Anspruch 9, wobei die Lichtquelle (6) Licht (L) mit einer vorgebbaren oder vorgegebenen Wellenlänge und/oder zeitweise in den Lichtleiter (5) einkoppelt.

11. Vorrichtung (1, 10, 100) nach Anspruch 10, wobei die Lichtquelle (6) Licht (L) mit einer Wellenlänge von zumindest 400 nm und/oder für eine Zeitdauer von einer Minute bis zehn Minuten in den Lichtleiter (5) eingekoppelt.

12. Verfahren (V) zur Herstellung der Vorrichtung (1, 10, 100) nach einem der vorhergehenden Ansprüche zur Verwendung bei der Behandlung von Bakterien in einer Körperhöhle (2) umfasst zumindest folgende Schritte:
- Einlegen eines textilen Flächengebildes (3, 30) in eine Körperhöhle (2) mit einer nach außen hin offenen Körperhöhlenöffnung (2.3), wobei das textile Flächengebilde (3, 30) zumindest eine Gas-freisetzende Substanz (M) umfasst, **dadurch gekennzeichnet, dass** die zu behandelnde Körperhöhle (2) ein Gehörgang ist, wobei das Verfahren ferner die folgenden Schritte umfasst:
- Einlegen eines Dichtungselements (4) im Bereich der Körperhöhlenöffnung (2.3) und Abdichten der Körperhöhlenöffnung (2.3) und
- Hindurchführen eines Lichtleiters (5) durch das Dichtungselement (4),
- Einkopplung von Licht (L) in den Lichtleiter (5) und Transport des Lichts (L) durch das Dichtungselement (4) hindurch und Einbringen des Lichts (L) auf oder in das textile Flächengebilde (3, 30).

## Claims

1. Device (1, 10, 100) comprising a textile sheet material (3, 30) for use as a therapeutic agent in treating an inflammatory disease in a body cavity (2),
the textile sheet material (3, 30) being provided with at least one gas-releasing substance (M) which releases an antibacterial gas (G) on activation by light (L),
**characterized in that** the body cavity (2) to be treated is an auditory canal,
the device (1, 10, 100) further comprising a sealing element (4) for sealing the auditory canal and a light guide (5) passed through the sealing element (4),
light (L) being introducible into the textile sheet material (3, 30) and/or the textile sheet material (3, 30) being irradiatable by light (L) by means of the light guide (5),
the at least one gas-releasing substance (M) being activatable by said light (L) and releasing an antibacterial gas (G).

2. Device (1, 10, 100) according to Claim 1, wherein the gas-releasing substance (M) releases a toxic gas (G) on activation.

3. Device (1, 10, 100) according to Claim 1 or 2, wherein the gas-releasing substance (M) releases carbon monoxide and/or nitric oxide on activation.

4. Device (1, 10, 100) according to any of the preceding claims, wherein the gas-releasing substance (M) is a carbonyl complex of a transition metal or a nitrosyl complex.

5. Device (1, 10, 100) according to any of the preceding claims, wherein the gas-releasing substance (M) is at least Mn₂(CO)₁₀.

6. Device (1, 10, 100) according to any of the preceding claims, **characterized by** a nonwoven fabric (3.1).

7. Device (1, 10, 100) according to Claim 6, wherein the nonwoven fabric (3.1) is a nanostructured hybrid nonwoven.

8. Device (1, 10, 100) according to any of the preceding claims, wherein the sealing element (4) is formed of wax.

9. Device (1, 10, 100) according to any of the preceding claims, further comprising at least one light source (6) which is connectable or connected to the light guide (5) for coupling light (L) into the light guide (5).

10. Device (1, 10, 100) according to Claim 9, wherein the light source (6) couples light (L) into the light guide (5) at a specifiable or specified wavelength and/or temporarily.

11. Device (1, 10, 100) according to Claim 10, wherein the light source (6) couples light (L) into the light guide (5) at a wavelength of at least 400 nm and/or for a period of one minute to ten minutes.

12. Method (V) for producing the device (1, 10, 100) according to any of the preceding claims for use in treating bacteria in a body cavity (2) comprises at least the following steps:
- inserting a textile sheet material (3, 30) into a body cavity (2) having an outwardly open body cavity opening (2.3), the textile sheet material (3, 30) comprising at least one gas-releasing substance (M),
**characterized in that** the body cavity (2) to be treated is an auditory canal, the method further comprising the following steps:
- inserting a sealing element (4) in the region of the body cavity opening (2.3) and sealing the body cavity opening (2.3) and
- passing a light guide (5) through the sealing element (4),
- coupling light (L) into the light guide (5) and transferring the light (L) through the sealing element (4) and introducing the light (L) onto or into the textile sheet material (3, 30).

## Revendications

1. Dispositif (1, 10, 100) comprenant une structure plane textile (3, 30) destinée à être utilisée comme moyen thérapeutique dans le traitement d'une maladie inflammatoire dans une cavité corporelle (2),
la structure plane textile (3, 30) étant pourvue d'au moins une substance libérant un gaz (M), laquelle, lors de son activation par la lumière (L), libère un gaz antibactérien (G), **caractérisé en ce que** la cavité corporelle (2) à traiter est un conduit auditif,
le dispositif (1, 10, 100) comprenant en outre un élément d'étanchéité (4) destiné à étanchéifier le conduit auditif et un guide de lumière (5) amené à passer au travers de l'élément d'étanchéité (4),
de la lumière (L) pouvant être introduite dans la structure plane textile (3, 30) et/ou la structure plane textile (3, 30) pouvant être irradiée par la lumière (L), au moyen du guide de lumière (5),
ladite au moins une substance libérant un gaz (M) étant activable par cette lumière (L) et libérant un gaz antibactérien (G).

2. Dispositif (1, 10, 100) selon la revendication 1, dans lequel la substance libérant un gaz (M) libère, lors de son activation, un gaz toxique (G).

3. Dispositif (1, 10, 100) selon la revendication 1 ou 2, dans lequel la substance libérant un gaz (M) libère, lors de son activation, du monoxyde de carbone et/ou du monoxyde d'azote.

4. Dispositif (1, 10, 100) selon l'une des revendications précédentes, dans lequel la substance libérant un gaz (M) est un complexe carbonylé d'un métal de transition ou un complexe nitrosylé.

5. Dispositif (1, 10, 100) selon l'une des revendications précédentes, dans lequel la substance libérant un gaz (M) est au moins du Mn₂(CO)₁₀.

6. Dispositif (1, 10, 100) selon l'une des revendications précédentes, **caractérisé par** une nappe de tissu (3.1).

7. Dispositif (1, 10, 100) selon la revendication 6, dans lequel la nappe de tissu (3.1) est une nappe hybride nanostructurée.

8. Dispositif (1, 10, 100) selon l'une des revendications précédentes, dans lequel l'élément d'étanchéité (4) est constitué de cire.

9. Dispositif (1, 10, 100) selon l'une des revendications précédentes, comprenant en outre au moins une source de lumière (6) qui peut être reliée ou est reliée au guide de lumière (5) pour l'injection de lumière (L) dans le guide de lumière (5).

10. Dispositif (1, 10, 100) selon la revendication 9, dans lequel la source de lumière (6) injecte de la lumière (L) dans le guide de lumière (5) à une longueur d'onde qui peut être spécifiée ou qui est spécifiée et/ou temporairement.

11. Dispositif (1, 10, 100) selon la revendication 10, dans lequel la source de lumière (6) injecte de la lumière (L) dans le guide de lumière (5) à une longueur d'onde d'au moins 400 nm et/ou pendant une durée d'une minute à dix minutes.

12. Procédé (V) pour la fabrication du dispositif (1, 10, 100) selon l'une des revendications précédentes, destiné à être utilisé dans le traitement des bactéries dans une cavité corporelle (2), comprend au moins les étapes suivantes :
- mise en place d'une structure plane textile (3, 30) dans une cavité corporelle (2) présentant une ouverture de cavité corporelle (2.3) ouverte vers l'extérieur, la structure plane textile (3, 30) comprenant au moins une substance libérant un gaz (M), **caractérisé en ce que** la cavité corporelle (2) à traiter est un conduit auditif, le procédé comprenant en outre les étapes suivantes :
- mise en place d'un élément d'étanchéité (4) dans la zone de l'ouverture de la cavité corporelle (2.3) et l'étanchéification de l'ouverture de la cavité corporelle (2.3), et
- le passage d'un guide de lumière (5) au travers de l'élément d'étanchéité (4),
- l'injection de lumière (L) dans le guide de lumière (5) et le transport de la lumière (L) au travers de l'élément d'étanchéité (4) et l'introduction de la lumière (L) sur ou dans la structure plane textile (3, 30).
